# EUROPEAN PATENT SPECIFICATION

(11) **EP 1 725 656 B1**
(45) Date of publication and mention of the grant of the patent: **02.10.2013**
(21) Application number: 05714574.0
(22) Date of filing: 04.03.2005
(51) Int. Cl.: C12N 5/0775

(54) **SERUM-FREE SUSPENSION CULTURE SYSTEM FOR MESENCHYMAL PROGENITOR CELLS**
SERUMFREIES SUSPENSIONSKULTURSYSTEM FÜRMESENCHYM-VORLÄUFERZELLEN
SYSTEME DE CULTURE EN SUSPENSION EXEMPTE DE SERUM POUR DES CELLULES SOUCHES MESENCHYMATEUSES

(30) Priority: 05.03.2004 US 549910 P
(43) Date of publication of application: 29.11.2006
(73) Proprietor: Davies, John E., Toronto, Ontario M6R 1J4 (CA); Baksh, Dolores, Mississauga, Ontario L4W 3H8 (CA); Zandstra, Peter, Toronto, Ontario M6G 2K8 (CA)
(72) Inventor: Davies, John E., Toronto, Ontario M6R 1J4 (CA); Baksh, Dolores, Mississauga, Ontario L4W 3H8 (CA); Zandstra, Peter, Toronto, Ontario M6G 2K8 (CA)
(74) Representative: Moore, Derek
(86) International application number: PCT/CA2005/000332
(87) International publication number: WO 2005/085428

(56) References cited:
- WO-A-00/27996
- WO-A-02/086104
- WO-A-2005/045011
- WO-A1-02/086104
- PITTENGER ET AL: "Multilineage Potential of Adult Human Mesenchymal Stem Cells" SCIENCE, [Online] XP002443155 Retrieved from the Internet: URL:http://www.sciencemag.org/feature/data /983855.dtl>
- EMERSON S G: "EX VIVO EXPANSION OF HEMATOPOIETIC PRECURSORS, PROGENITORS, AND STEM CELLS: THE NEXT GENERATION OF CELLULAR THERAPEUTICS" BLOOD, W.B. SAUNDERS, PHILADELPHIA, VA, US, vol. 87, no. 8, 15 April 1996 (1996-04-15), pages 3082-3088, XP002909281 ISSN: 0006-4971
- BAKSH DOLORES ET AL: "Soluble factor cross-talk between human bone marrow-derived hematopoietic and mesenchymal cells enhances in vitro CFU-F and CFU-O growth and reveals heterogeneity in the mesenchymal progenitor cell compartment" BLOOD, vol. 106, no. 9, November 2005 (2005-11), pages 3012-3019, XP002443262 ISSN: 0006-4971
- BAKSH D. ET AL: 'Adult human bone marrow-derived mesenchymal progenitor cells are capable of edhesion-independent survival and expansion.' EXP.HEMATOL. vol. 31, no. 8, August 2003, pages 723 - 732, XP009086938
- PITTENGER M.F. ET AL: 'Multilineage Potential of Adult Human Mesenchymal Stem Cells.' SCIENCE., [Online] vol. 284, no. 5411, April 1999, pages 143 - 147, XP000867221 Retrieved from the Internet: <URL:www.sciencemag.org/feature/data/983855 .shl>
- KALLOS M.S. ET AL: 'Large-scale expansion of mammalian neutral stem cells: a review.' MED. BIOL. ENG COMPUT. vol. 41, no. 3, May 2003, pages 271 - 282, XP001536379
- MINGUELL J.J. ET AL: 'Mesenchymal Stem Cells.' EXP BIOL MED. vol. 226, no. 6, 2001, pages 507 - 520, XP002429905
- GRONTHOS S. ET AL: 'The Growth Factor Requirements of STRO-1 Positive Human Bone Marrow Stromal Precursors Under Serum-Deprived Conditions in Vitro.' BLOOD. vol. 85, no. 4, February 1995, pages 929 - 940, XP002402196
- LENNON D.P. ET AL: 'A chemically defined medium suports in vitro proliferation and maintains the osteochondral potential of rat marrow-derived mesenchymal stem cells.' EXP. CELL RES. vol. 219, no. 1, July 1995, pages 211 - 222, XP000981537
- RUOSLAHTI E ET AL: "NEW PERSPECTIVES IN CELL ADHESION RGD AND INTEGRINS", SCIENCE (WASHINGTON D C), vol. 238, no. 4826, 1987, pages 491-497, ISSN: 0036-8075
- BOURDON M A ET AL: "TENASCIN MEDIATES CELL ATTACHMENT THROUGH AND RGD-DEPENDENT RECEPTOR", JOURNAL OF CELL BIOLOGY, vol. 108, no. 3, 1989, pages 1149-1156, ISSN: 0021-9525
- PEREIRA RUTH F ET AL: "Marrow stromal cells as a source of progenitor cells for nonhematopoietic tissues in transgenic mice with a phenotype of osteogenesis imperfecta", PROCEEDINGS OF THE NATIONAL ACADEMY OF SCIENCES OF THE UNITED STATES OF AMERICA, vol. 95, no. 3, 3 February 1998 (1998-02-03), pages 1142-1147, ISSN: 0027-8424
- RUOSLAHTI E: "RGD AND OTHER RECOGNITION SEQUENCES FOR INTEGRINS", ANNUAL REVIEW OF CELL AND DEVELOPMENTAL BIOLOGY, ANNUAL REVIEWS, US, vol. 12, 1 January 1996 (1996-01-01), pages 697-715, XP000944583, ISSN: 1081-0706, DOI: 10.1146/ANNUREV.CELLBIO.12.1.697 ISBN: 978-0-8243-3113-9

## Description

### Field of the Invention

This invention is in the field of cell biology. More particularly, the invention relates to the culturing of mesenchymal progenitor cells, which upon differentiation give rise to various connective tissues including bone and cartilage.

### Background of the Invention

WO02/086104 published October 31, 2002 teaches that mammalian, and particularly human, mesenchymal progenitor cells (MPCs) capable of colony forming unit - fibroblast (CFU-F) and osteoblast (CFU-O) development can successfully be cultured and expanded at relatively large scale under non-static and non-contact suspension culture conditions where the cells are present as non-adherent individual cells, and not as either large clumps or as confluent layers. The survival and expansion of such cells can be improved by supplementing the culturing medium with various growth factors and agents, such as interleukin-3 (IL-3) and stem cell factor (SCF). Accordingly, the technique of non-static, non-adherent, suspension culturing is established as a valuable method by which relatively large populations of MPCs can be generated either for further research or for medical purposes such as cell-based therapies including tissue engineering. This technique also yields subpopulations of cells having a unique phenotype that is not revealed during adherence-based culturing of mesenchymal cell populations.

It is an object of the present invention to provide an improved method for the culturing of progenitor cells, including mesenchymal progenitor cells.

### Summary of the Invention

It has now been found that progenitor cells can be cultured, i.e., maintained and expanded, by non-static and non-contact suspension culture conditions where the cells are present as non-adherent individual cells, in culturing medium that is serum-deprived. Thus, there is provided a process for culturing a progenitor cell population, comprising the step of culturing an input population of progenitor cells by non-contact, non-adherent, stirred suspension in serum-deprived medium. The cultured progenitor cell population comprises, and is optionally enriched for, non-hematopoietic progenitor cells. Preferably, the cultured progenitor cell population comprises, and is optionally enriched for, progenitor cells having a CD45- phenotype. Preferably, the input progenitor cell population comprises, and is optionally enriched for, mesenchymal progenitor cells. The serum-deprived medium is supplemented with one or more agents, including growth factors and/or hematopoietic progenitor cells, to foster and/or enhance the survival and/or expansion of the cultured progenitor cells.

There is disclosed a method for producing a population of non-hematopoietic cells, including mesenchymal cells, comprising the step of obtaining progenitor cells cultured in accordance the culturing method of present invention, and then growing the cultured progenitor cells under conditions conducive to the differentiation thereof into mesenchymal cells and/or tissue. By this process, there is provided such mesenchymal tissues as bone and cartilage.

In another of its aspects, the present invention provides a progenitor cell population enriched for cells having a CD45⁻CD123⁺ phenotype. In a related aspect, the present disclosure describes the use of such a cell population in the production of mesenchymal tissues.

### Brief Description of the Drawings

In drawings which illustrate by way of example only a preferred embodiment of the invention,

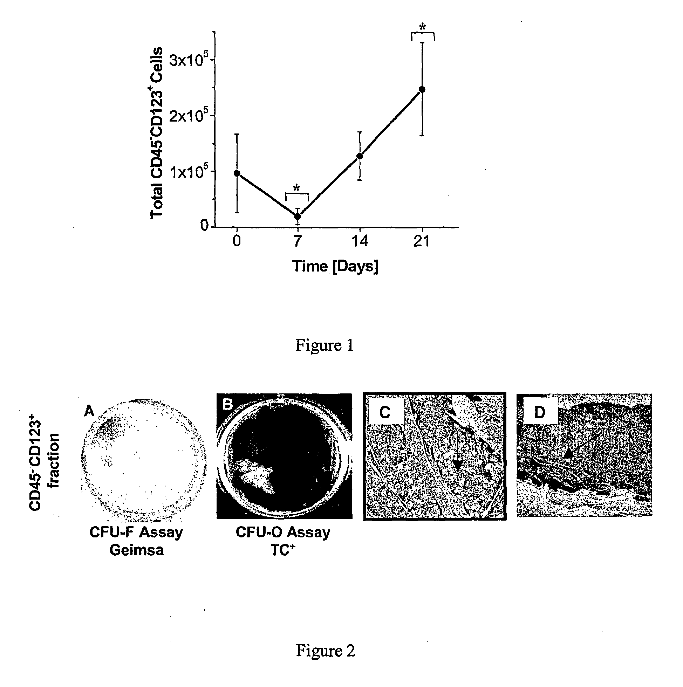

Figure 1 shows the total number of CD45⁻CD123⁺ cells generated in serum-free suspension culture conditions supplemented with 100 ng/ml SCF and 20 ng/ml IL3. Dual fluorescence labeling for CD45 and CD123 was used to track the percentage of CD45⁻CD123⁺ cells in suspension cultures initiated with CD45⁻ cells. These values were used to calculate the total number of CD45⁻CD123⁺cells generated throughout suspension culture. Statistical significance (p<0.05) is denoted by *. Each data point represents the mean ± SD (n=3).
Figure 2 shows CFU-F and CFU-O development from CD45⁻CD123⁺ suspension-derived cells. CD45⁻CD123⁺ sorted cells (grown in CD45⁻ stirred suspension cultures) were plated in CFU-F and CFU-O assays following 7 days of suspension culture. (A) CFU-F assays were stained with Giemsa to visualize discrete colonies of fibroblastic cells while (B) CFU-O assays were incubated with tetracycline (TC), to visualize newly formed bone nodules (seen as white areas) under UV fluorescence microscopy. Representative scanning electron micrographs reveal (C) cement line matrix deposition (arrow) and (D) collagen mineralization (arrow) derived from CD45⁻CD123⁺ sorted cells grown in CFU-O assay conditions (F.W. for C and D: 53 µm and 21 µm, respectively). (Note: CFU-F and CFU-O assays were initiated with 1x10³ cells/cm²).

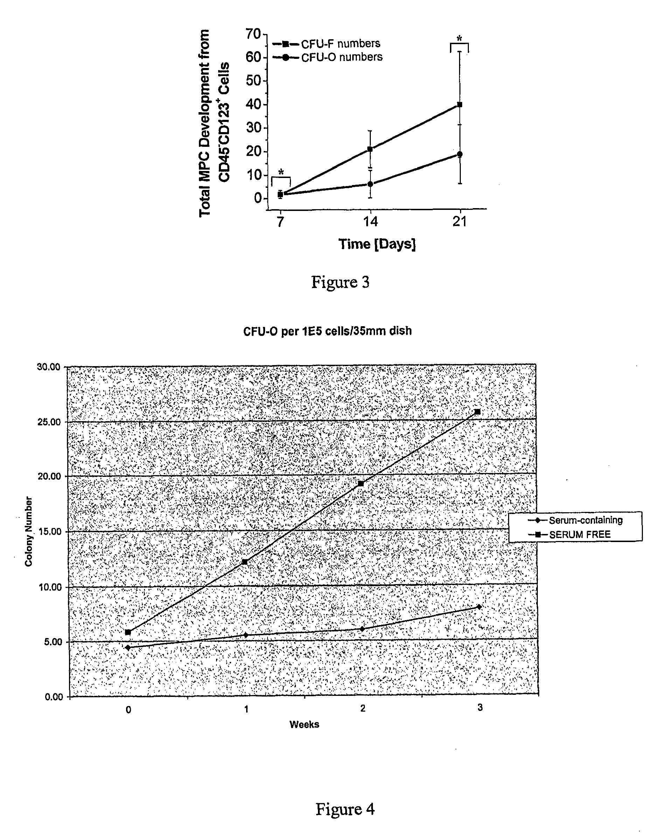

Figure 3 shows the total number of CFU-F and CFU-O generated from CD45⁻CD123⁺ cells grown in suspension cultures initiated with CD45⁻ cells. The absolute number of CFU-F and CFU-O at each time point was determined by multiplying their frequency detected in CFU-F and CFU-O assays, respectively, by the total number of CD45⁻CD123⁺ cells present in the culture at each time point. Each symbol represents the mean ± SD (n=3). A statistically significant difference (p<0.05) was observed in the yield of both CFU-F and CFU-O between Day 7 and 21 (denoted by *). Note: there was no CFU-F and CFU-O detected from CD45⁻CD123⁺ cells isolated from input (Day 0) bone marrow-derived cells; and
Figure 4 shows a comparison of the CFU-O colonies counted in 35mm dishes seeded with 1E5 cells in both serum-containing and serum-free conditions. It can be seen that the number of CFU-O colonies is greater for each time point than that in the serum-containing condition. Furthermore, the rate of increase in the number of CFU-O colonies counted is greater in the serum-free condition.

### Detailed Description of the Invention

In the present invention, input cell populations comprising non-hematopoietic progenitor/stem cells, including CD45- progenitor cells and particularly including mesenchymal progenitor cells, are cultured by non-static, non-adherent, suspension culturing in a culturing medium that is not supplemented with serum.

The non-static, non-adherent, suspension culturing technique is designed most generally to culture cells under conditions that essentially prevent the cultured cells from adhering to the surface of the culturing vessel, and also prevent the cultured cells from growing for any significant time period in direct contact with each other as agglomerated cell masses, as cell masses nucleated on a synthetic scaffold, or as confluent cell layers. To achieve such non-static, non-adherent, culturing conditions, a given input cell population can be cultured under conditions that impart movement within the liquid culturing medium, for instance using agitation such as by shaking or stirring, or using fluidizing techniques such as percolation, aspiration, and the like, using equipment and conditions established for this purpose. To minimize adherence during culturing, the bioreactor suitably is treated, e.g., siliconized, to inhibit cell adherence to the internal surfaces of the bioreactor. Thus, conditions of non-static, non-adherent culturing are designed to maintain cultured cells in suspension as individual cells during their culturing.

The agitation source is typically a mechanical agitation means that either introduces movement either directly within the culturing medium or externally through the surface on which the culturing vessel is placed. The non-static, non-adherent, conditions can be introduced by stirring means, which can include a magnetized stirring paddle that is placed in the bioreactor and is induced by external magnetizing means. Alternatively, the agitation can be introduced by other means that include fluidized culturing beds, which introduce agitation through the movement of appropriate culturing atmosphere through the cell population.

It will be appreciated that the magnitude of the agitation will be that which is sufficient to maintain the cells in suspension substantially as individual, expandable cells with desired circulation of culturing nutrients, but insufficient to introduce shear forces that will result in cell lysis. A stirring speed of about 40 rpm is suitable.

The culture system can be configured such that i) the impeller is placed in the middle of the bioreactor at a 90° angle to the solution surface to maintain axial flow, ii) a constant mixing speed of 40 rpm is used to maintain the cells in suspension and iii) the agitator is positioned three quarters of the way down the vessel to ensure uniform mixing.

Other non-static, non-adherent, suspension culturing are described for instance in W002/0861 04 published October 31, 2002.

With the non-static, non-adherent culturing approach, the present method introduces a three-dimensional environment for cell expansion, and raises exponentially the volume of the environment within which progenitor cells can be expanded, thereby offering increases not only in the number of cells that can be expanded at any given time but also accelerating the rate at which the cells can be expanded over time. In the present method, suitable culturing volumes can range from the milliliters to many litres, for instance from about 0.1 L to more than 200L, e.g., 0.5L to 100L, such as 1L to 10L.

In accordance with the present invention, the input population of progenitor or stem cells is cultured in a liquid nutrient medium that is essentially free of serum as a supplement, such as fetal bovine serum, horse serum and human serum, and otherwise comprises the components essential for survival and expansion particularly of mesenchymal progenitor cells. Thus, the culturing medium used in the present invention is one that lacks the serum component responsible in other systems for introducing unknown and occasionally infectious agents.

A variety of liquid nutrient media, such as basal nutrient media, are suitable for use in the present method. The progenitor cells can be cultured under non-static, non-adherent, conditions in the presence of chemically defined media available commercially for the culturing of progenitor cells, including the StemSpan™ Serum-Free Expansion medium available commercially from StemCell Technologies in Vancouver, British Columbia. Alternatively, the culturing medium can be Dulbecco's Modified Eagle's Medium (DMEM), Iscove's Modified Dulbecco's Medium, McCoy's Modified SA Medium, Minimum Essential Medium Eagle, RPMI 1640 Medium, Ham's F12 and mixtures thereof, for instance as described in US 6,617,161 issued September 9, 2003. The suitability of a particular medium or media mixture can be determined readily by culturing the input population of mesenchymal progenitor cells in the presence of the chosen medium under non-static, non-adherent, conditions as herein described. Suitable media are those which sustain and more preferably expand the population of input mesenchymal progenitors.

The media in which the cells are cultured is supplemented with one or more growth factors, or cellular sources thereof, which enhance survival and expansion of the mesenchymal progenitors. Where the survival or expansion/proliferation of mesenchymal progenitor cells is desired, the culturing media supplemented with interleukin-3 (IL-3) or IL-3 and SCF. Preferably, the mesenchymal progenitor cell culturing media is supplement with both SCF and IL-3. Other growth factors can also be added, including fibroblast growth factor (FGF). As noted in the examples herein, the addition of FGF was found not to enhance expansion of the mesenchymal progenitors to a statistically significant extent. Moreover, the addition of platelet derived growth factor (PDGF) was found to have a negative impact on mesenchymal progenitor expansion. Thus, the mesenchymal progenitor cell culturing medium can be devoid of PDGF supplement, and can be optionally further devoid of FGF supplement.

For the expansion of other non-hematopoietic progenitor cell types, such as those giving rise to neuronal tissue, the culturing media can be supplemented with PDGF in an amount effective to support their growth and expansion. More generally, the present disclosure contemplates the culturing progenitor cells in the presence of such agents, including growth factors and cytokines, as are established for purposes of providing the general survival and proliferative signals to the target progenitor cell type. Such agents are readily identified, under the present non-static, non-adherent, serum-free culturing conditions using the smaller scale conditions described in the examples.

Thus, preferably, the present method of an input population comprising mesenchymal progenitor cells by non-static, non-adherent, suspension culturing comprises culturing the cells in the presence of nutrient medium that is essentially free from serum supplement and comprises an amount of IL-3 or a combination of IL-3 and SCF effective to enhance survival and/or expansion thereof.

When incorporated in the culturing medium for mesenchymal progenitors or other progenitors, SCF and IL-3 are present at concentrations desirably of about 20ng/mL e.g., about 1-100ng/mL for IL-3 and about 100ng/mL e.g., 10-1,000ng/mL for SCF, or a variation thereof that yields improvement in expansion of the mesenchymal progenitors.

Culturing of the input population can be conducted under conditions otherwise established for this purpose. Temperatures suitably lie within the physiological range. The culturing atmosphere is desirably the appropriate blend of O₂ and CO₂, e.g., humidified 5% CO₂ in air. Culturing time periods that are suitable are reflected in the examples herein, will depend to some extent on the scale of the culture and culturing conditions, and will general entail periods e.g., about one week or more such as two or three weeks or more, by which an expansion of mesenchymal progenitors should be evident.

The present culturing method most desirably is applied to an input progenitor cell population that is obtained without any prior step of selection based on cell adherence or anchorage dependence, which is more common in the art, and which in itself is believe to impart certain phenotypic traits to other progenitor cell populations that have been cultured in accordance with prior art methods.

The cells cultured in accordance with the present method, i.e., the input population, comprise, and may be enriched for, non-hematopoietic (NHP) progenitor and stem cells. The NHP cells include the mesenchymal progenitor cells (MPCs) and progenitor cells that give rise to other non-blood cell types, and thus as a class, give rise to a wide variety of cell types and tissues of therapeutic interest, including nerve tissue, connective tissue, muscle, tendon/ligament, bone, cartilage, adipose tissue and vascular endothelium.

Preferably, the present culturing method is applied to expand progenitor cells having a CD45- phenotype.

Preferably, the present culturing method is applied to expand mesenchymal progenitor cells within the input progenitor cell population.. This input population is characterized by the ability to give rise, under conditions conducive to such differentiation, to cell types and tissues that include osteocytes, chondrocytes, adipocytes and other mesenchymal cell types, which give rise to such tissues as bone, cartilage, muscle including cardiac tissue, tendon and adipose. Phenotypically, the mesenchymal progenitors typically are CD45-.

Suitable sources of input cell populations include bone marrow stroma, umbilical cord including Wharton's jelly, umbilical cord blood and placental blood, placenta, peripheral blood, skin, adipose tissue and muscle. The non-hematopoietic progenitor cells available from these sources can be extracted using techniques well established in the art.

The input cell population can be enriched for non-hematopoietic progenitor cells before culturing. This can be achieved as established, for instance using enrichment media such as Rosette-SepTM (StemCell Technologies) and a Ficoll spin, or more concertedly by the FACS-based sorting procedure in combination with antibodies to markers on the cell types to be subtracted. Such antibodies, and procedures and devices for performing such subtractions are commercially available. It is possible for instance to subtract from the extracted cells those that are phenotypically CD45+, and which thus are of the hematopoietic progenitor cell type.

Desirably, and in accordance with an embodiment of the invention, the input population of mesenchymal progenitor cells further comprises cells of the CD45+ phenotype, e.g., cells of the hematopoietic lineage. Thus, in the present method, it is not essential that that the extracted progenitor cells are first sorted to enrich for the CD45- mesenchymal progenitors. Rather, the extracted progenitor cell population can be subjected as a whole to non-static, non-adherent, culturing under serum-deprived conditions. It has been found that non-mesenchymal CD45+ progenitors in the mixed input population can contribute factors useful in the survival and expansion of the mesenchymal progenitors.

The present method is particularly useful to enrich for progenitor cells having a CD45-/CD123+ phenotype. The present disclosure thus further describes an isolated population of mesenchymal progenitor cells that is enriched for, and desirably consists essentially of, cells having a CD45-/CD123+ phenotype.

It will be appreciated that such isolated cell populations can be obtained by selection of the desired cell phenotype from the cultured, expanded population such as by FACS-based cell sorting.

The cell populations generated from the input progenitor cell populations are useful medically in various therapies designed, for instance, to repair or regenerate tissue of any type into which these populations can differentiate. Cells cultured in accordance with the present method are particularly useful for this purpose, because they are relative small in size (about 6 microns) and therefore generally smaller than for instance red blood cells which because of their larger size are unable to penetrate endogenous sites to which their delivery might be desired. Moreover, the cells cultured by the present invention have the demonstrated ability to survive and expand as, and thus are more likely to be deliverable as, individual cells, and not as clumps or aggregations that tend to form when using cells cultured in contact with tissue culture plastic. Thus, for medical use, the expanded mesenchymal progenitor cell population can be formulated for delivery to the site at which differentiation is desired, using any delivery vehicle that is physiologically tolerable to both the recipient and to the viability of the cells formulated therein. Particular formulations and suitable delivery vehicles are known in the art, and will be apparent from the nature of the intended therapy. Desirably, the formulation further contains agents, such as growth factors and cytokines, which enhance the viability and/or the differentiation of the administered cells.

The present disclosure also describes a method of using specifically differentiated cells for therapy comprising administering the specifically differentiated cells to a patient in need thereof. It further describes for the use of genetically engineered multipotent stem cells to selectively express an endogenous gene or a transgene, and for the use of the progenitor cells either per se or in expanded form in vivo for transplantation/administration into an animal to treat a disease. The cells can be used to engraft a cell into a mammal comprising administering autologous, allogeneic or xenogenic cells, to restore or correct tissue specific structural or other function to the mammal. The cells can be used to engraft a cell into a mammal, causing the differentiation in vivo of cell types, and for administering the progenitor or differentiated cells into the mammal. The cells, or their in vitro or in vivo differentiated progeny, can be used to correct a genetic disease, degenerative disease, neural, or cancer disease process. They can be used to produce gingiva-like material for treatment of periodontal disease. They could be used to enhance muscle such as in the heart. A genetically engineered progenitor cell, or its differentiated progeny, can be used to treat a disease with CNS deficits or damage. Further the progenitor cells, or neuronally related differentiated progeny, can be used to treat a disease with neural deficits or degeneration including among but not limited to stroke, Alzheimer's, Parkinson's disease, Huntington's disease, AIDS associated dementia, spinal cord injury, metabolic diseases effecting the brain or other nerves.

The progenitor cells, or cartilage differentiated progeny, can be used to treat a disease of the joints or cartilage such as cartilage tears, cartilage thinning, and osteoarthritis. Moreover, the cells or their osteoblast differentiated progeny can be used to treat bone disorders and conditions, such as bone fractures, osteoarthritis, bone voids caused by surgery or tumors for tissue regeneration in osteoporosis, Paget's disease, and osteomyelitis.

As noted, inducing the progenitor cells, and their expanded equivalents, to differentiate is achieved using techniques established in the art, which vary according to the differentiated cell type desired. For differentiation to osteoblasts, progenitors can be cultured for about 14-21 days in culturing medium comprising supplements such as dexamethasone, β-glycerophosphate and ascorbic acid, and optionally including various bone growth factors. The presence of osteoblasts can be confirmed by Von Kossa staining, or antibodies against a bone cell marker such as bone sialoprotein, osteonectin, osteopontin and osteocalcin.

For differentiation into chondroblasts, the progenitors can be grown in serum-free DMEM supplemented with TGF-β in suspension pellet culture, for about 14 days or more.

To induce adipocyte differentiation, dexamethasone and insulin, or media supplemented with approximately 20% horse serum, can be used. Adipocyte differentiation can be detected by examination with light microscopy, staining with oil-red, or detection of lipoprotein lipase (LPL), adipocyte lipid-binding protein (αP2), or peroxisome proliferator-activated receptor gamma (PPAR). Adipocytes can be used for the treatment of Type II diabetes, and in reconstructive or cosmetic surgery, e.g., for breast reconstruction after mastectomy, or for reshaping tissue lost as a result of other surgery.

To induce skeletal muscle cell differentiation, progenitor cells can be treated with 5-azacytidine in expansion medium for a period, and then transferred to LTC medium. Differentiation can be confirmed by detecting sequential activation of Myf-5, Myo-D, Myf-6, myogenin, desmin, skeletal actin and skeletal myosin, either by immunohistochemistry or Western blot analysis. Smooth muscle cells can also be induced by culturing progenitors in serum-free medium, without growth factors, supplemented with high concentrations of platelet-derived growth factor (PDGF). Terminally differentiated smooth muscle cells can be identified by detecting expression of desmin, smooth muscle specific actin, and smooth muscle specific myosin by standard methods. Cardiac muscle differentiation can be accomplished by adding basic fibroblast growth factor (bFGF) to the standard serum-free culture media without growth factors.

It will thus be appreciated that the progenitor cells of the present disclosure, their expanded equivalents and their differentiated progeny can be used in cell replacement therapy and/or gene therapy to treat a variety of conditions.

It will be appreciated that cells provided by the present disclosure can be used to produce tissues or organs for transplantation. Oberpenning, et al. (Nature Biotechnology (1999) 17: 149-155) reported the formation of a working bladder by culturing muscle cells from the exterior canine bladder and lining cells from the interior of the canine bladder, preparing sheets of tissue from these cultures, and coating a small polymer sphere with muscle cells on the outside and lining cells on the inside. The sphere was then inserted into a dog's urinary system, where it began to function as a bladder. Nicklason, et al., Science (1999) 284: 489-493, reported the production of lengths of vascular graft material from cultured smooth muscle and endothelial cells. Other methods for forming tissue layers from cultured cells are known to those of skill in the art (see, for example, Vacanti, et al., U. S. Patent No. 5,855,610). These methods can be especially effective when used in combination with cells of the present invention, which have a broad range of differentiation.

The cells can be provided as frozen stocks, alone or in combination with prepackaged medium and supplements for their culture, and can be additionally provided in combination with separately packaged effective concentrations of appropriate factors to induce differentiation to specific cell types. Alternately, the cells can be provided as frozen stocks containing cells induced to differentiate by the methods described herein above.

The expanded progenitor cells and particularly the expanded mesenchymal progenitors can be utilized in bone therapy. To this end, the cells can be delivered as such or together with a suitable matrix, such as a scaffold, liquid or gelatinous material, by injection or applied as a paste to a site at which bone formation is desired. Alternatively, the cells can be placed ex vivo in a differentiation environment, exemplified by the CFU-O conditions described herein, and then transplanted to the intended site when their differentiation to bone tissue has reached an appropriately mature stage.

### Examples

The current study implemented rigorous examination of a variety of candidate soluble factors, under serum-deprived conditions, to arrive at optimized soluble factor combination(s) that would influence the growth of a MPC population in suspension. To accomplish this, a factorial design approach (Box et al., 1978) was implemented to screen for combinations of important soluble factors that have a positive effect on MPC expansion. Specifically, we evaluated the effects of fibroblast growth factor (FGF), platelet-derived growth factor (PDGF), stem cell factor (SCF) and interleukin-3 (IL3) and their respective 2⁴ possible combinations on total cell, CFU-F and CFU-O expansion. FGF and PDGF were chosen for these studies as they have been shown to stimulate both the recruitment and growth of mesenchymal cells (detected as CFU-F) in both serum and serum-free conditions (Bianchi et al., 2003;Tsutsumi et al., 2001;Kuznetsov et al., 1997a;Gronthos and Simmons, 1995b;Hock and Canalis, 1994;Hirata et al., 1985b). IL3 and SCF, although not typically used to stimulate the growth of mesenchymal cells, were investigated here based on previous results demonstrating that the combination of SCF and IL3 (albeit in the presence of serum) resulted in a significant expansion of CFU-F and CFU-O following suspension culture. These analyses revealed that IL3 was the most effective stimulator on the survival of MPCs and when combined with SCF, supported expansion of MPCs in suspension. Importantly, these findings demonstrate the unique ability to grow mesenchymal progenitor cell types in serum-free suspension culture conditions.

### Materials and Methods

### Human bone barrow derived-cell isolation

Human bone marrow aspirates were obtained from the iliac crest of normal donors (n=3; 2 female donors: ages 37 and 38 yrs. old; 1 male donor: 25 yrs old). The bone marrow aspirates were fractionated on a Ficoll-Paque™ gradient (Sigma, St. Louis, MO) as previously reported. The recovered cells were counted and plated at 1x10⁶ cells per 4 ml StemSpan™ (serum-free) medium (StemCell Technologies, Vancouver, BC, CA) per well of a 6-well plate.

### Serum-Free Culture

### CFU-F Assay

CFU-F potential of suspension-derived cells (and input cells) was determined by plating aliquots (1,000 cells/cm²) of test cells in MesenCult™ medium (StemCell Technologies) in 24-well or 35 mm polystyrene tissue culture plates at 37°C with 5% humidified CO₂. The medium was exchanged every 3-4 days. After 14 days of incubation, the cultures were terminated and stained for α-napthyl acetate esterase activity (Sigma, Oakville, ON, CA). Following, cultures were stained with Giemsa modified solution (Sigma) to visualize cell nuclei and cytoplasm. Colonies of fibroblasts containing >50 α-napthyl acetate esterase-negative cells were then enumerated. Of note, CFU-F assays very rarely contained cells or colonies that were α-napthyl acetate esterase positive.

### CFU-O Assay

CFU-O potential was determined by plating aliquots (1,000 cells/cm²) of test cells from days 0, 7, 14 and 21 onto 24-well or 35 mm polystyrene tissue culture plates and incubating at 37°C with 5% humidified CO₂. Osteogenic medium comprised α-MEM (Medium Preparation Services, University of Toronto, CA), 15% fetal bovine serum (StemCell Technologies) and 10% antibiotics/antifungal solution (167 units/mL penicillin G, 50µg/mL gentamicin, 0.3µg/mL amphotericin B) supplemented with 50µg/mL L-ascorbic acid (Sigma) and 3.5mM sodium β-glycerophosphate (Sigma). After 33 days of culture, 9 µg/ml tetracycline (Sigma) in ddH₂O was added to the cultures in the last re-feed prior to termination at 35 days as a technique to visualize, via UV-fluorescence microscopy, the newly mineralized matrix deposited by the cells. The cultures were then terminated after a total culture period of 35 days and subsequently, colonies of osteoblasts (CFU-O) labeling positive for tetracycline were enumerated. Some CFU-O cultures were also stained with alkaline phosphatase followed by staining with von Kossa. Representative cultures were prepared for SEM analysis to visualize the matrix formed.

### Factorial Design

A two-level factorial design matrix (Box et al., 1978) was constructed (Table 4.1) to evaluate the effects of FGF, PDGF, SCF and IL3 and their respective 2⁴ possible combinations on total cell, CFU-F and CFU-O expansion. In this factorial design study, optimal (high) or a zero dose levels for PDGF and FGF was chosen based on previous studies (Gronthos and Simmons, 1995b;Wang et al., 1990), which reported that PDGF and FGF at a concentration of 20-100 ng/mL stimulated the expansion of human mesenchymal stem/progenitor cells as assayed by their capacity to form CFU-F. A dose level of 20 ng/ml was chosen for SCF and IL3 in these studies.

### Results

### IL3 and SCF support total cell, CFU-F and CFU-O expansion in serum-free conditions

To develop a serum-free suspension culture configuration to more thoroughly characterize the specific role played by soluble factors on MPC growth in suspension, FGF, PDGF, SCF and IL3, either alone or in combination, were screened for their capacity to support the survival and expansion of total cells, CFU-F and CFU-O in suspension. At 7, 14 and 21 days, the total number of cells were calculated and CFU-F and CFU-O assays were initiated with cells derived from each condition. The greatest expansion in the outcomes studied (i.e., total cell, CFU-F and CFU-O expansion) was achieved in the SCF+IL3 and SCF+IL3+PDGF treatment conditions, while minimal growth was achieved in the single factor groups. Importantly, the results obtained in the serum-free system were comparable to those described in the serum-containing cultures.

### Analysis of SCF, IL3, PDGF and FGF interactive effects

Univariate analysis of variance (Univariate ANOVA) and linear regression analyses on the output responses (i.e., total cell, CFU-F and CFU-O expansion) at day 21 were performed using SPSS 11.0. Single factor positive effects on total cell, CFU-F and CFU-O expansion were identified only in conditions where SCF or IL3 were added. PDGF and FGF had no significant (positive) impact on total cell, CFU-F or CFU-O growth. Interestingly, the combination of FGF+SCF+IL3 resulted in the expansion of total cell, CFU-F and CFU-O (>1-fold), relative to Day 0 values. However, there were no statistically significant differences observed between the SCF+IL3 and SCF+IL3+FGF treatment groups, suggesting that the presence of FGF does not further stimulate the MPC expansion in suspension beyond levels attainable in the combined presence of SCF and IL3. Notably, cells derived from the combination of all growth factors (SCF+IL3+PDGF+FGF) failed to give rise to CFU-O after 7 days of suspension culture.

The addition of PDGF to the SCF+IL3 combination resulted in a statistically significant negative β-value (-0.764) for CFU-O expansion; however, the fold expansion calculated was >5-fold.

### The importance of IL3 on the expansion of MPCs

It is evident that cytokine combinations containing both SCF and IL3 resulted in significant positive effects on the outcomes studied. To specifically determine the individual contribution of each of these growth factors on the tested outcomes, interaction plots were constructed. These suggested an interaction effect between SCF and IL3 on total cell expansion, but that SCF alone does not significantly affect total cell expansion. Conversely, the presence of 20 ng/ml IL3 resulted in positive effects on total cells.

### Further Exemplification

The example above demonstrates that cells capable of forming CFU-F and CFU-O can be propagated in non-contact serum-free conditions. The development of a serum-free suspension culture system promotes examination of the effects of specific endogenous and exogenous factors on the growth of MPCs. Therefore, in the present study, the specific role of IL3 on MPC expansion in suspension was examined. One possible mechanism of IL3 action is through direct binding to CD123 receptors (IL3α receptor) present on MPCs themselves. Alternatively, IL3 could also impact MPC total cell, CFU-F and CFU-O expansion by acting indirectly through binding and acting on hematopoietic cells (CD45⁺ cells), whose surface expression of the CD 123 receptor is well documented. This receptor binding and the resulting signaling cascades could potentially elicit secondary effects and release of soluble factors by the IL3-stimulated hematopoietic cells, which subsequently stimulate MPC. In order to directly test the hypothesis that IL3 acts on non-hematopoietic cells capable of CFU-F and CFU-O development, CD45⁻ cells, in the absence of CD45⁺ cells, were cultured in suspension in the presence of 100 ng/ml SCF and 20 ng/ml IL3. Flow cytometric analysis of the sorted CD45⁻ cell population revealed the emergence of significant numbers of CD45⁻CD123⁺cells after 21 days of suspension culture (relative to Day 0 values). Cell sorting on the CD45⁻-sorted population demonstrated that a subpopulation of CD45⁻CD123⁺ cells had the capacity to give rise to CFU-F and CFU-O throughout culture expansion. These findings reveal that CFU-F capable MPCs are phenotypically heterogeneous. The yield of CFU-F and CFU-O from the CD45⁻CD123⁺ cell population increased throughout the study period, suggesting that IL3 interacts with CD123 receptor expressing cells resulting in their proliferation. Additionally, these studies unexpectedly revealed a significantly lower number of detectable CFU-F and CFU-O when MPCs were grown in the absence of CD45⁺ cells. These results suggest that hematopoietic cells may secrete factor(s) that affect the growth of MPCs, potentially in response to the addition of exogenous cytokines, such as IL3 and SCF. Therefore, we can conclude that MPC expansion in suspension is modulated by hematopoietic cells (via their endogenous secretion of factors) and by the exogenous supplementation of IL3 to the culture medium.

More particularly, it has been demonstrated that growth of adherent-derived mesenchymal stem cells (MSCs), detected as CFU-F, is regulated by a number of specific soluble growth factors (Gronthos and Simmons, 1995b). PDGF-BB, EGF and bFGF have been shown to have the greatest capacity to support colony growth under serum-deprived conditions (Bianchi et al., 2003;Kuznetsov et al., 1997a;Gronthos and Simmons, 1995b;Hirata et al., 1985b). However, PDGF and FGF had no significant impact on the growth of MPCs in suspension, while IL3 had the most potent affect on the growth of CFU-F and CFU-O. In normal hematopoiesis, IL3 stimulates cell cycle progression and differentiation, while inhibiting apoptosis of hematopoietic cells. Specifically, IL3 plays an important role in the growth and differentiation of CD34⁺ progenitor cells into basophils and mast cells, myeloid-derived dendritic cells DCs and nonmyeloid-derived DCs [reviewed in (Martinez-Moczygemba and Huston, 2003)]. Cells responsive to the action of IL3 express CD123 (IL3α receptor) and are typically of the hematopoietic lineage (i.e., expressing CD45) (Munoz et al., 2001;Huang et al., 1999;de Groot et al., 1998). In normal human bone marrow, CD123 has been shown to be expressed in 0.27% of the total nucleated cells. However, CD123 displays differential expression levels in various cell compartments of bone marrow (Munoz et al., 2001). For example, ∼53% of the precursor compartment (CD34⁺ cells), ∼63% of the myeloid compartment (CD34⁺CD33⁺C19⁻ cells) and 0% of the lymphoid compartment (CD34⁺CD33⁻CD19⁺CD10⁺) express CD123 in normal human bone marrow (Munoz et al., 2001). To date, no evidence exists that suggests bone marrow contains non-hematopoietic cells with mesenchymal developmental potential that express CD123. However, Pittenger et al. (1999) (in supplemental notes) document that adhesion-dependent, bone marrow-derived mesenchymal stem cells express CD123. These studies did not describe the functional role of CD123 and we have shown that culture of similar adherent-derived cells was not affected by IL3 supplementation. Furthermore, the cells grown under contact culture conditions lacked any detectable expression of CD123, suggesting that IL3 is not a mitogen for growth of adherent-derived MSCs.

Notably, maintenance in the numbers of suspension-cultured MPCs capable of forming CFU-F and CFU-O were observed in direct response to IL3, suggesting that IL3 may influence MPC growth either through direct interaction with CD123 receptors on MPCs or indirectly by first binding to CD123 on hematopoeitic cells which release soluble factors that subsequently promote MPCs survival and/or growth. In the absence of IL3, suspension-cultured MPCs failed to survive. Therefore, the current study was designed to test the hypothesis that IL3 directly interacts with a suspension-grown MPC population expressing CD123, leading to their proliferation in suspension. It is well documented that IL3 transduces signals in hematopoietic cells (CD45⁺ cells) (McAdams et al., 1996;Brandt et al., 1994). Therefore, to remove the complications and potential interactions between hematopoeitic (CD45⁺ cells) and non-hematopoietic cells test the hypothesis of direct IL3-MPC interaction, non-hematopoietic cells (CD45⁻ cells) were isolated and grown in serum-free suspension culture medium supplemented with SCF and IL3. Flow cytometry was used to kinetically track the CD123 expression on a sorted CD45⁻ cell population. The results revealed a competitive emergence of a CD45⁻CD123⁺ cell population in direct response to the addition of IL3 and SCF. The CFU-F and CFU-O developmental potential was determined from a purified CD45⁻CD123⁺ subfraction isolated from the sorted CD45⁻ cell population. Specifically, ∼35% and ∼21% of the total attainable CFU-F and CFU-O, respectively, could be recovered from the CD45⁻CD123⁺ cell subpopulation. These results indicate the existence of phenotypic heterogeneity in the putative mesenchymal stem compartment. Of particular significance, these studies also revealed that co-culture with hematopoietic cells results in an increased number of CFU-F and CFU-O, suggesting that endogenously produced factors from hematopoietic cells also influence MPC growth in suspension. This work raises the possible unique regulatory role that both IL3 and endogenously secreted factors from hematopoietic cells have on the growth of MPCs in suspension and promotes the use of this heterogeneic cell culture (i.e., incorporating both non-hematopoietic and hematopoietic input cells) suspension system to more fully enhance the output of colony forming MPCs.

### Materials & Methods

### Serum-free suspension cultures initiated with unsorted, CD45⁺, CD45⁻cells

To explicitly study the regulation of MPC growth in suspension and eliminate confounding influences attributed to the presence of hematopoietic cells, the CD45⁻ cell fraction of BM MNCs were isolated. BM MNCs from normal donors (n=3) were incubated with saturating concentrations (1:100 dilution) of conjugated mouse IgG_{l,κ} anti-human CD45-FITC (BD Biosciences, Canada) and both CD45 negative and positive cells were sorted on a Beckman-Coulter EPICS Cell Sorter (CA) at a rate of 2000-3000 cells/seconds at 10.5 psi to 99.5% purity. Stirred suspension cultures (n=3, for each group) were initiated with unsorted, CD45⁻ and CD45⁺ cells and cultured in StemSpan™ medium (Product#: 09650, Stem Cell Technologies, Va, BC, Canada) supplemented with 20 ng/ml IL3 and 100 ng/ml SCF (n=3) (No Cytokine suspension cultures were performed in parallel for each group). StemSpan™ medium contains all the necessary serum-replacement components (i.e., albumin, rh insulin, human transferrin, 2-mercaptoethanol, L-glutamine and Iscove's MDM). At 7, 14 and 21 days, the total number of cells were counted and test cell populations from each condition were initiated in CFU-F and CFU-O assays (n=3, in triplicate). Flow cytometry was used to track the relative percentages of CD45⁺ and CD45⁻ suspension cell populations during the 21-day study period.

### Flow cytometric sorting of suspension-derived CD45⁻CD117⁺ and CD45⁻CD123⁺ cells

On days 7, 14 and 21, cells from suspension cultures (No Cytokine and SCF+IL3 treatment groups) that were initiated with CD45⁻ cells on Day 0 (input) were incubated with saturating concentrations of conjugated mouse IgG_{l,κ} anti-human CD45-FITC and CD123-PE (IL3α receptor) and CD117-PE (SCF receptor) (BD Biosciences, Canada) to recover populations of CD45⁻CD123⁺ and CD45⁻CD117⁺ cells on a Beckman-Coulter EPICS Cell Sorter at a rate of 2000-3000 cells/seconds at 10.5 psi to 99.5% purity. The CFU-F and CFU-O developmental potential of these cell population was assayed.

### Statistical Analysis

Pair-wise statistical comparisons were made, as indicated, by the Student's t-Test. A difference was considered significant at p<0.05.

### Results

### Flow cytometric tracking of unsorted and CD45⁻ cell populations

CD45⁻ cells were initiated in stirred suspension cultures in the presence of 100 ng/ml SCF and 20 ng/ml IL3 in serum-free conditions. At each time point (including Day 0), the expression of CD45 was tracked using flow cytometry in suspension cultures initiated with CD45⁻ cells to ensure that the cell population maintained a relatively homogenous CD45⁻ phenotype. Flow cytometric analysis revealed that the suspension cultures initiated with CD45⁻ cells maintained a >97% CD45⁻ phenotype throughout the 21-day culture period (Day 0: 99% CD45⁻ cells). On average, ∼1% of the cells expressed the CD45 antigen.

### The presence of CD45⁺ cells and their endogenously secreted factors influence CFU-F and CFU-O expansion in suspension

At 7, 14 and 21 days, test cells were removed from stirred suspension cultures, which were initiated with unsorted, CD45⁺ and CD45⁻ bone marrow-derived cells grown under serum-free culture conditions supplemented with 100 ng/ml SCF and 20 ng/ml IL3. The total number of viable cells was calculated and the CFU-F and CFU-O developmental potentials from these populations were assayed. Cells isolated from suspension cultures initiated with unsorted and CD45⁻ sorted cells gave rise to discrete colonies of CFU-F and CFU-O. No significant difference in the CFU-F colony sizes from these cell fractions (unsorted vs. CD45⁻ sorted cells) was observed at any time points studied. An average CFU-F colony size of 7.2 ± 1.9 mm and 6.6 ± 1.2 mm was calculated from Day 7 derived cells from the unsorted and CD45⁻sorted cell population, respectively. No CFU-F or CFU-O development was observed from the CD45⁺-sorted subfraction of cells grown in suspension.

Calculating the fold expansion in total cells, CFU-F and CFU-O, relative to input numbers, revealed that the unsorted cell group [containing both CD45⁻ and CD45⁺ cells (>60% of unsorted cell fraction were CD45⁺)] resulted in a higher fold increase in total, CFU-F and CFU-O cells compared to the numbers obtained from the CD45⁻ sorted cell population at all time points studied. Statistically significant differences (p<0.05) were calculated between the unsorted and CD45⁻ sorted cell groups with respect to the fold expansion in total cells, CFU-F and CFU-O at various time points. Minimal CFU-F and CFU-O growth was observed in the absence of CD45⁺ cells. Surprisingly, however, on average, a 58 ± 11 % increase in CFU-F and CFU-O developmental potential was observed when CD45⁻ cells were cultured in the presence of CD45⁺ cells, over the study period, suggesting an interaction exists between the hematopoietic and non-hematopoietic cell fractions.

### IL3 promotes for the growth of CD45⁻CD123⁺ cells

Incubation with SCF+IL3 resulted in a significant increase in the percentage of cells expressing CD123 (i.e., between Day 7 and Day 21). The relative expression of CD45⁻CD123⁺ cells increased from 3.5 ± 1.3% (Day 7) to 8.9 ± 2.3% by Day 21 (4.7 ± 1.2% of the CD45⁻ Day 0 input cells expressed CD123).

Flow cytometry was used to calculate the total number of CD45⁻CD123⁺ cells that were generated throughout suspension culture (Fig. 1). A decrease in the number of CD45⁻CD123⁺ cells was observed from Day 0 to Day 7, which may be attributed to apoptosis in those cells not supported under these culture conditions. However, after Day 7, CD45⁻CD123⁺ cells a significant (p<0.05) increase in number and an ∼12-fold increase in total CD45⁻CD123⁺ cells was observed by Day 21, relative to Day 7 values.

CD45⁻ cells were also evaluated for their expression of CD 117 (SCF receptor). Flow cytometric analysis revealed that <1% of the total CD45⁻ population was CD117⁺. Furthermore, CD45⁻CD117⁺ cells, isolated on Day 7 of suspension culture, failed to give rise to CFU-F and CFU-O. Additionally, the existence of a CD45⁻CD123⁺CD117⁺ and/or CD45⁻CD123⁺CD117⁻ cell population was not detected in these studies. These results suggest that SCF does not directly interact with suspension-cultured MPCs isolated in the CD45⁻ cell fraction. However, based on the observation that SCF enhances the activity of IL3 other mechanisms by which SCF supports the growth of MPCs may be involved (i.e., through hematopoietic cells and their release of endogenous factors that target MPCs). Collectively, these results demonstrate the ability of IL3 to enhance expansion of MPCs in suspension culture, and suggest that its action may occur via direct interaction with CD123 expressing MPCs and/or indirectly through interaction with CD123⁺ hematopoietic and elicitation of subsequent soluble factors, which then directly interact with MPCs to facilitate suspension culture expansion.

### CFU-F and CFU-O developmental potential of CD45⁻CD123⁺ suspension-derived cells

The developmental potential of CD45⁻CD123⁺ cells (isolated from CD45⁻ suspension cell cultures) was tested in CFU-F and CFU-O assays on Day 7, 14 and 21. The frequency of CFU-F and CFU-O in the CD45⁻CD123⁺ cell population was determined from these assays after 7, 14 and 21 days of suspension culture. By Day 7 of suspension culture, ∼1 in 10⁴ CD45⁻CD123⁺ cells had the capacity to form CFU-F and CFU-O (Fig 2). There were no CFU-F and CFU-O detected from input (Day 0) bone marrow-derived CD45⁻CD123⁺ cells at the cell seeding dilution used in the respective functional assays (i.e., 1x10³ cells/cm²).

Tracking the total yield of CFU-F and CFU-O over the 21-day culture period recovered in the CD45⁻CD123⁺ cell fraction revealed a statistically significant (p<0.05) increase in the numbers of both CFU-F and CFU-O by Day 21 relative Day 7 values (Fig. 3). However no statistically significant differences were detected between the numbers of CFU-F and CFU-O obtained at each of the time points examined, suggesting that a CD45⁻CD123⁺ cell population may contain a unique cell type responsive to IL3, which has the capacity to give rise to both CFU-F and CFU-O.

### Discussion

It was demonstrated above that it was possible to culture MPCs in serum-free suspension culture conditions, and IL3 was revealed to be an important modulator for the growth of MPCs. A possible mechanism by which IL3 exerts its action on MPCs is indirectly through hematopoietic cells (CD45⁺ and their subsequent release of factors that then specifically target MPCs. However, to explore the potential action of IL3 directly acting on MPCs, resulting in their growth, CD45⁻ cells (devoid of all CD45⁺ cells) were cultured in serum-free suspension culture conditions supplemented with SCF and IL3. Flow cytometric analysis confirmed that >97% of the cells maintained a CD45⁻ phenotype throughout suspension culture. Incubation with SCF and IL3 resulted in a significant increase in the percentage and total number of CD45⁻CD123⁺ cells by Day 21, suggesting that the growth of CD45⁻CD123⁺ cells is supported under these conditions. Importantly, we demonstrated that the CD45⁻ cell fraction comprised a small percentage of CD123⁺ cells (range: ∼4.0% to ∼11% at 7 and 21 days, respectively) that demonstrated the capacity to form CFU-F and CFU-O after 7, 14 and 21 days of suspension culture. There were no CFU-F or CFU-O detected in the CD45⁻CD123⁺ cell fraction on Day 0, suggesting that the cell giving rise to CFU-F and CFU-O may be at a very low frequency in bone marrow or, perhaps MPCs "acquire" this phenotype after extended time in suspension culture.

Various embodiments of the present invention having been thus described in detail by way of example, it will be apparent to those skilled in the art that variations and modifications may be made without departing from the invention. The invention includes all such variations and modifications as fall within the scope of the appended claims.

## Claims

1. A process for culturing a progenitor cell population, comprising the step of culturing an input population of non-haematopoietic progenitor cells, by non-static non-adherent suspension in serum-deprived nutrient medium,
**characterised in that**,
the serum-deprived nutrient medium is supplemented with a factor selected from interleukin-3 or a combination of interleukin-3 and stem cell factor, and wherein said selected factor is a modulator for the expansion of the progenitor cells.

2. A process according to Claim 1, wherein stem cell factor is present at a concentration between 100ng/mL and 1000ng/mL.

3. A process according to Claim 1 or 2, wherein interleukin-3 is present at a concentration between 1 ng/mL and 100ng/mL.

4. A process according to any of the preceding Claims, wherein the input population comprises mesenchymal progenitor cells having a CD45- phenotype.

5. A process according to any of the preceding Claims, wherein said input population further comprises a population of CD45+ progenitor cells.

6. A process according to any one of the preceding Claims, wherein the input cell population is cultured for a period sufficient to expand the progenitor cell population therein.

7. A suspension culture comprising an isolated population of mesenchymal progenitor cells having a CD45-/CD123+ phenotype and a serum-deprived culture medium, wherein the serum-deprived culture medium is supplemented with a factor selected from interleukin-3 or a combination of interleukin-3 and stem cell factor, and wherein said selected factor is a modulator for the expansion of the progenitor cells.

## Patentansprüche

1. Verfahren zur Kultivierung einer Vorläuferzellpopulation, welche den Schritt der Kultivierung einer Eingangspopulation aus nicht-hämatopoetischen Vorläuferzellen durch eine nicht statische, nicht anhaftende Suspension in serumfreiem Nährmedium umfasst,
**dadurch gekennzeichnet, dass**
das serumfreie Nährmedium mit einem Faktor angereichert ist, der wahlweise aus Interleukin-3 oder einer Kombination von Interleukin-3 und Stammzellfaktor besteht, und wobei der gewählte Faktor ein Regler der Expansion der Vorläuferzellen ist.

2. Verfahren nach Anspruch 1, wobei der Stammzellfaktor in einer Konzentration zwischen 100 ng/ml und 1000 ng/ml vorliegt.

3. Verfahren nach Anspruch 1 oder 2, wobei Interleukin-3 in einer Konzentration zwischen 1 ng/ml und 100 ng/ml vorliegt.

4. Verfahren nach einem der vorangegangenen Ansprüche, wobei die Eingangspopulation mesenchymale Vorläuferzellen vom Phänotyp CD45- umfasst.

5. Verfahren nach einem der vorangegangenen Ansprüche, wobei die Eingangspopulation weiter eine Population von CD45+ Vorläuferzellen umfasst.

6. Verfahren nach einem der vorangegangenen Ansprüche, wobei die Eingangszellpopulation für ausreichend lange Zeit kultiviert wird, um die enthaltene Vorläuferzellpopulation zu expandieren.

7. Eine Suspensionskultur, die eine isolierte Population aus mesenchymalen Vorläuferzellen mit dem Phänotyp CD45-/CD123+ und ein serumfreies Kulturmedium umfasst, wobei das serumfreie Kulturmedium mit einem Faktor angereichert ist, der wahlweise aus Interleukin-3 oder einer Kombination von Interleukin-3 und Stammzellfaktor besteht, und wobei der gewählte Faktor ein Regler der Expansion der Vorläuferzellen ist.

## Revendications

1. Procédé de culture d'une population de cellules souches, comprenant l'étape de la culture d'une population d'entrée de cellules souches non hématopoïétiques, par une suspension non statique et non adhérente dans un milieu nutritif privé de sérum,
**caractérisé en ce que**,
le milieu nutritif privé de sérum est complété par un facteur choisi à partir d'interleukine-3, ou une combinaison d'interleukine-3 et de facteur de cellules souches, et dans lequel ledit facteur choisi est un modulateur pour l'expansion des cellules souches.

2. Procédé selon la revendication 1, dans lequel le facteur de cellule souche est présent à une concentration comprise entre 100 ng/ml et 1000 ng/ml.

3. Procédé selon la revendication 1 ou 2, dans lequel l'interleukine-3 est présente à une concentration comprise entre 1 ng/ml et 100 ng/ml.

4. Procédé selon l'une quelconque des revendications précédentes, dans lequel la population d'entrée comprend des cellules souches mésenchymateuses présentant un phénotype CD45-.

5. Procédé selon l'une quelconque des revendications précédentes, dans lequel ladite population d'entrée comprend en outre une population de cellules souches CD45+.

6. Procédé selon l'une quelconque des revendications précédentes, dans lequel la population de cellules d'entrée est mise en culture pendant une durée suffisante pour développer la population de cellules souches qui s'y trouve.

7. Culture en suspension, comprenant une population isolée de cellules souches mésenchymateuses présentant un phénotype CD45-/CD123+ et un milieu de culture privé de sérum, dans lequel le milieu de culture privé de sérum est complété par un facteur choisi à partir d'interleukine-3, ou une combinaison d'interleukine-3 et de facteur de cellules souches, et dans lequel ledit facteur choisi est un modulateur pour l'expansion des cellules souches.
